# EUROPEAN PATENT SPECIFICATION

(11) **EP 0 755 659 B1**
(45) Date of publication and mention of the grant of the patent: **05.04.2000**
(21) Application number: 95305299.0
(22) Date of filing: 28.07.1995
(51) Int. Cl.: A61B 19/00

(54) **Surgical instrument holder**
Halter für chirurgisches Instrument
Support d'instrument chirurgical

(43) Date of publication of application: 29.01.1997
(73) Proprietor: Putman, John Michael, Dallas, Texas 75246 (US)
(72) Inventor: Putman, John Michael, Dallas, Texas 75246 (US)
(74) Representative: Lawrence, Malcolm Graham

(56) References cited:
- EP-A- 0 293 760
- WO-A-94/22377
- WO-A-97/04713
- US-A- 5 184 601

## Description

This invention relates generally to the art of universal positioning devices, and in particular to a manually releasable clamp assembly for holding a surgical instrument such as an endoscope during a surgical procedure.

In the performance of surgery and related procedures, sterile operating conditions are maintained by a surgical drape which covers the patient and the operating table. The surgical procedure is performed through a slit or preformed fenestration which is aligned with a desired surgical site. It is sometimes necessary to support and stabilize an instrument such as an endoscope or laparoscope at an elevated position above the patient for long periods of time, with a portion of the instrument being inserted into the patient's abdominal cavity. The purpose of the endoscope instrument is to provide visual access into a body cavity, for example, the abdominal cavity, the knee, shoulder, bladder, uterus and bowel. A laparoscope is a type of endoscope that includes a viewing tube for insertion through the abdominal wall.

It is necessary to vary the position of the instrument from time to time according to the needs of the surgical procedure. During a laparoscopic cholecystectomy (gall bladder removal), for example, an endoscope is inserted into the upper abdominal cavity which is inflated and pressurized with carbon dioxide by an insufflating machine. The endoscope is guided through a trocar sheath which serves as an interface port through the abdominal wall. By sliding the endoscope up and down the port, or rotating it in a desired direction, a view of the internal organs can be obtained by a video camera which is attached to the endoscope, and the image is displayed on a video monitor.

The video camera also records the movement of other surgical instruments, for example, a grasper, a hook, a spatula, forceps and dissector, which are guided into and out of the abdominal cavity through one or more secondary surgical trocar sheaths. When the distal tip of the instrument appears on the video monitor, the surgeon guides it into place and controls its action and movement as displayed on the video monitor. It is usually necessary to re-position the endoscope from time to time to view the operative site so that the surgical instruments are positioned appropriately within the cavity to expose the organ or internal tissue for inspection, repair, dissection or excision.

The success of the laparoscopy procedure depends in part on the surgeon's ability to gauge spatial relationships as viewed on the video monitor, and in part on his ability to easily adjust or reposition the endoscope as the procedure progresses. During gall bladder removal, for example, it may be necessary to re-position the endoscope and hold it in a desired orientation as the gall bladder duct is sealed by a surgical clip. Additionally, it may be necessary to re-position the endoscope while using an electrocautery instrument to excise the gall bladder from the underside of the liver. After the gall bladder organ has been severed, it is removed through an exit port. It is then necessary to re-position the endoscope to an upper midline port so that the surgeon can correctly position and operate a grasper instrument through a secondary trocar port.

In some cases, operating room personnel manually hold the endoscope instrument in the desired position, and move it about according to the surgeon's instructions. The use of operating room personnel to support such instruments during an extended surgical procedure is unsatisfactory in that the assistant may be unable to maintain stability because of muscle fatigue, and find it necessary to change position at some critical or otherwise inconvenient time.

Support devices which are mountable onto an operating table have been used for holding surgical instruments such as endoscopes and retractors. Such equipment may be clamped onto the side rail of an operating table and are moved about from time to time as required by the surgical procedure. However, such devices may restrict access to the surgical site and have limited maneuverability. Moreover, because the side rails are closely located to the sterile operating field, certain instrument support positions are difficult to achieve. Generally, it is desirable to support surgical instruments in offset relation with respect to the operating table and side rails to allow a wide range of support positions.

Some rail-mounted support devices must be manually released from time to time to re-position instruments which are suspended above the sterile operating zone. It will be appreciated that in surgical procedures, time is of the essence, and delays associated with adjustment of support equipment prolong the procedure. Additionally, the presence of surgical support equipment within the sterile operating zone limits the surgeon's access to the patient during the procedure. Thus, it is generally desirable to limit the number of surgical support devices in and about the sterile zone so that the operating surgeon and his attendants will have clear and unrestricted access to the patient, and also will have a clear and unrestricted view of a video monitor.

During certain procedures, it may be desirable to impose or change a biasing force on the surgical instrument to stabilize its position within the abdominal cavity. It is awkward or impossible in some instances to apply such bias forces through instruments or apparatus which are mounted directly on the side rail. Thus, it is desirable to offset such equipment both laterally and vertically in the regions immediately surrounding the sterile zone of the operating table.

Accordingly there is a specific need for surgical instrument support apparatus which may be set up on a portable console outside of the sterile field for supporting a surgical instrument, such as an endoscope, in a desired viewing position and orientation within a body cavity, with the position of the instrument supporting apparatus including a quick-disconnect clamp for easily removing the instrument without disturbing the sterile field.

European Patent Application 293760 discloses a surgical instrument holding apparatus corresponding to the preamble of claim 1 and comprising in combination:
a coupling collar having a guide bore extending therethrough and a counterbore defining an inner wall;
latching means (groove in shaft) disposed on a shaft;
said shaft having an end portion which is insertable into the guide bore to an operative latched position;
quick disconnect means disposed on the coupling collar for releasably connecting the shaft to the coupling collar by engagement with the latching means, the quick disconnect means being radially deflectable to permit the shaft to be inserted and withdrawn from the guide bore; and,
clamp means disposed on the shaft for holding a surgical instrument.

The present invention provides a quick-disconnect instrument clamp for use in combination with the universal positioning arm of a portable support console. The instrument clamp holds and stabilizes a surgical instrument such as an endoscope during a surgical procedure, and is quickly and easily adjustable over a wide range of stable support positions. The quick-disconnect clamp is insertable into a coupling collar formed on a connecting portion of the positioning arm. The connecting portion can be shielded by a sterile drape so that the clamp can be manipulated without contamination. The quick-disconnect clamp can be made of a disposable material or a material suitable for resterilization. The clamp may be quickly and easily removed without disturbing the placement of the positioning arm or the sterile environment.

There is therefore provided a surgical instrument holding apparatus comprising a clamp for holding a surgical instrument, a shaft connected to the clamp, a coupling collar having a latch bore defining a retainer socket and including a latch receiver in the retainer socket, and the shaft including a proximal end portion and a distal end portion, the proximal end portion being insertable into the retainer socket and movable to a latched position with the distal end portion projecting out of the retainer socket when the proximal end portion is in the latched position, and a manually operable latch attached to the proximal end portion of the shaft for releasably connecting the shaft to the coupling collar, the latch including a latch head engageable with the latch receiver in the latched position, characterised in that:
the latch includes at least two resilient actuator arms, each arm havina a latch head, which actuator arms are radially spaced from the shaft, an end portion of each actuator arm projecting out of the retainer socket and being accessible for manual gripping outside of the coupling collar when the latch head is in the latched position, each actuator arm being movable radially inwardly relative to the shaft to permit the latch to be manually inserted into and withdrawn from the retainer socket, and being movable radially outwardly relative to the shaft for moving the latch head into latching engagement with the latch receiver.

Preferred embodiments are claimed in the dependent claims.

One way of carrying out the invention is described in detail below with reference to drawings which illustrate only one specific embodiment, in which :
FIG. 1 is a perspective view of a instrument clamp and portable console support apparatus shown set up adjacent to a surgical operating table;
FIG. 2 is a top plan view of the instrument clamp and portable console support apparatus of FIG. 1;

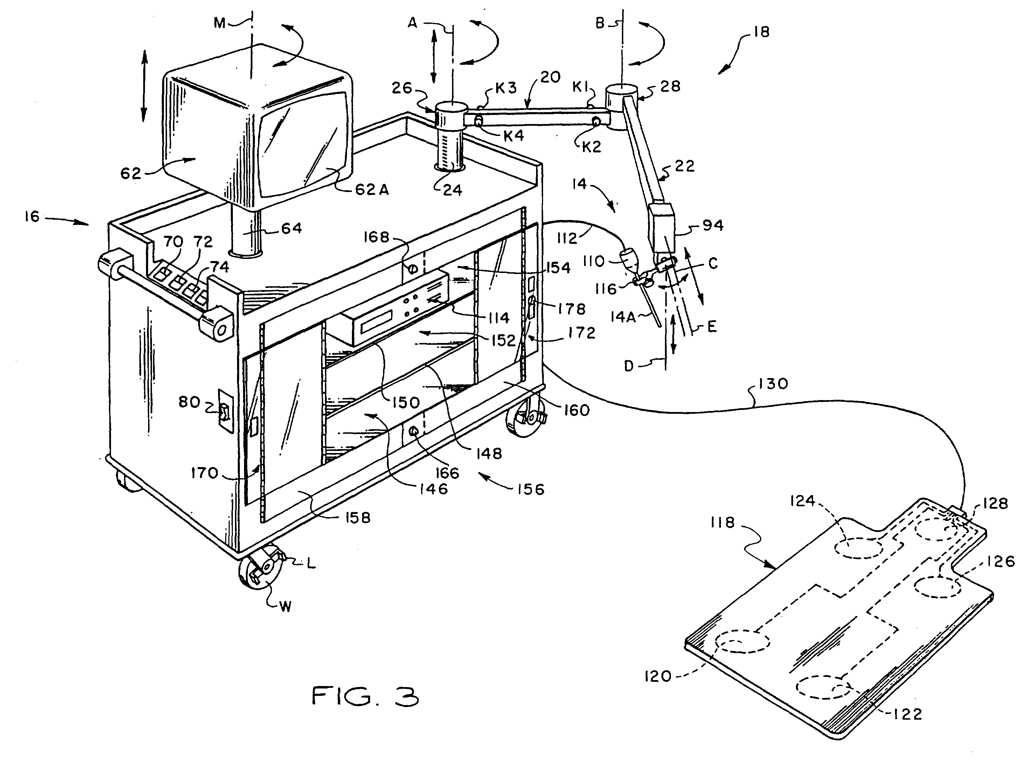

FIG. 3 is a perspective view of the instrument clamp and portable console support apparatus of FIG. 1;

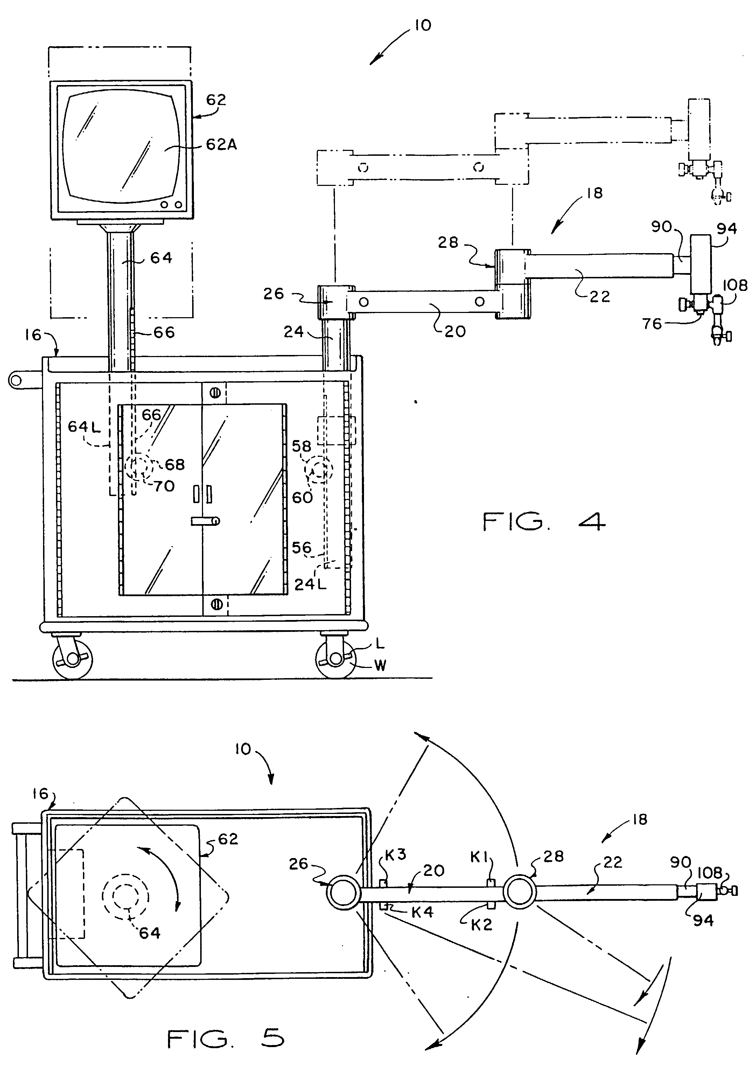

FIG. 4 is a front elevational view of the instrument clamp and positioning apparatus of FIG. 1;
FIG. 5 is a top plan view thereof;
FIG. 6 is an elevational view of the quick-disconnect instrument clamp of the present invention installed on an extendable support arm;
FIG. 7 is a perspective view of the adjustable coupling collar shown in FIG. 6;
FIG. 8 is a perspective side view of the quick-disconnect clamp shown in FIG. 6;
FIG. 9 is a cross-sectional view of the coupling collar taken approximately along the line 9-9 of FIG. 7;
FIG. 10 is a side elevational view, partially in section, of the quick- disconnect clamp and coupling collar assembly;
FIG. 11 is an enlarged sectional view showing the quick disconnect clamp in detented engagement with the coupling collar; and
FIG. 12 is a perspective view of an endoscope instrument installed in the quick-disconnect clamp of the present invention.

The surgical instrument support apparatus 10 of the present invention is particularly well suited for use in combination with a conventional surgical operating table 12 during the performance of abdominal, pelvic, joint, bladder, bowel and uterine surgery.

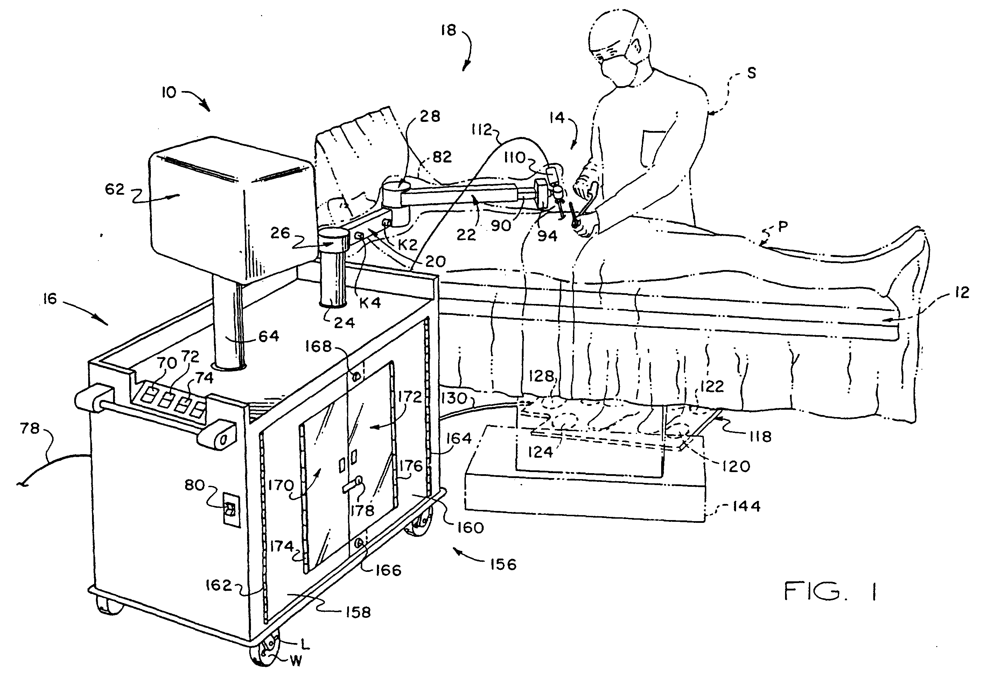

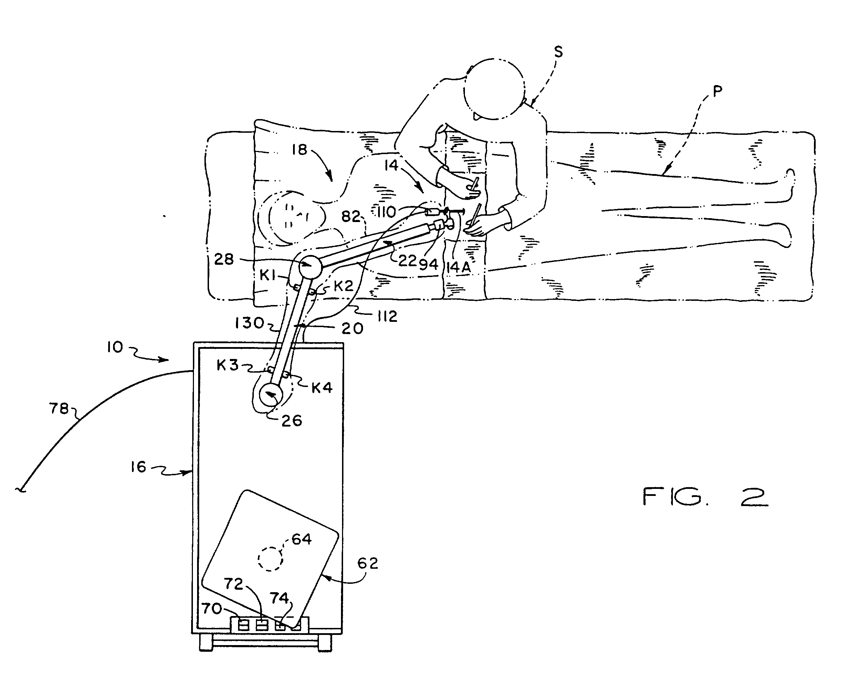

Referring now to FIGS. 1 and 2, the surgical instrument support apparatus 10 is shown set up adjacent to an operating table 12 for positioning an endoscope instrument 14 during a surgical procedure in the abdominal cavity of a patient P. Stable support is provided by a portable console 16 which is equipped with lockable wheels W for permitting rolling movement of the console 16 from one station to another. The portable console 16 is parked adjacent to the operating table 12, and is positioned substantially at a right angle with respect to the operating table 12 to provide standing room for attendants who assist the surgeon S. After the portable console 16 has been positioned correctly, its wheels W are locked by depressing the wheel lock arms L, and the surgical instrument support apparatus 10 is made ready by an attendant.

The endoscope instrument 14 is supported by an articulated arm assembly 18 which includes a first support section 20 and a second support section 22, rotatably supported by an upright support shaft 24. The support shaft 24 is movably mounted on the console 16 for extension and retraction in elevation. The first support section 20 is movably coupled to the upright support shaft 24 by a bearing assembly 26 which permits rotational movement of the first support section 20 relative to the support shaft 24. Likewise, the second support section 22 is rotatably coupled to the first support section 20 by a bearing assembly 28.

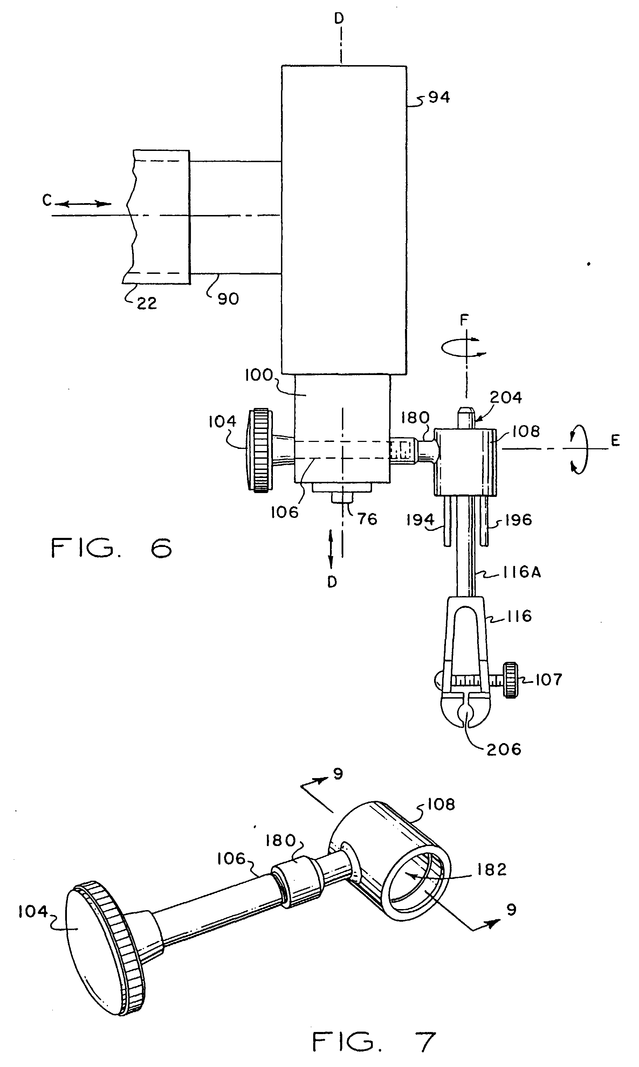

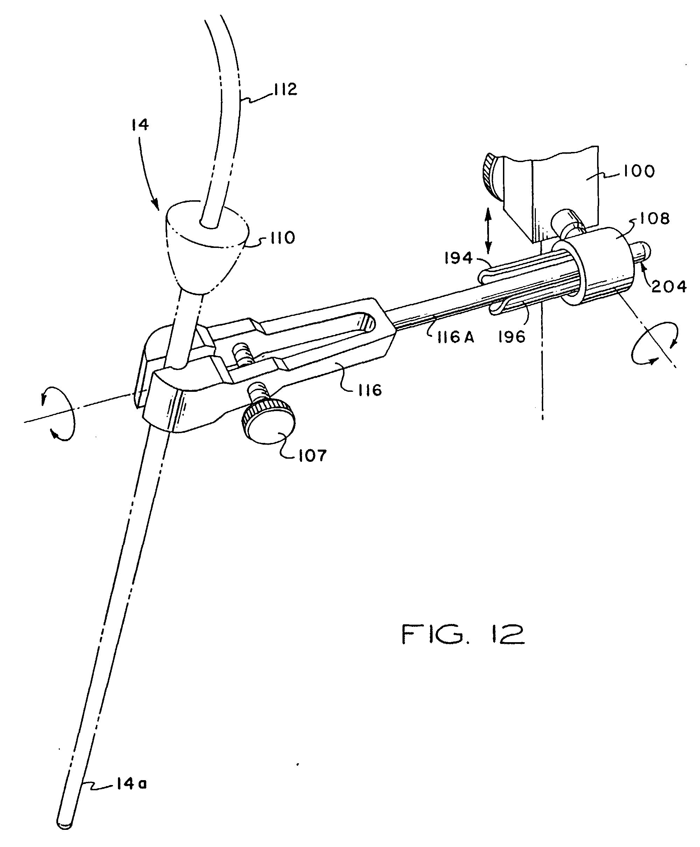

Referring to FIGS. 5-11, the endoscope instrument 14 is secured to a rotatable coupling collar 108 extending from the extendable positioning arm 100. The coupling collar 108 is secured to the distal end of the extendable positioning arm 100 by a screw clamp 104. The screw clamp 104 includes a threaded shaft 106 for engaging a threaded portion 180 formed on the outside wall of the coupling collar 108. In the relaxed position, the screw clamp 104 permits free rotation of the coupling collar 108 about the longitudinal axis E. The screw clamp 104 can then be tightened to maintain the coupling collar 108 in a preferred orientation.

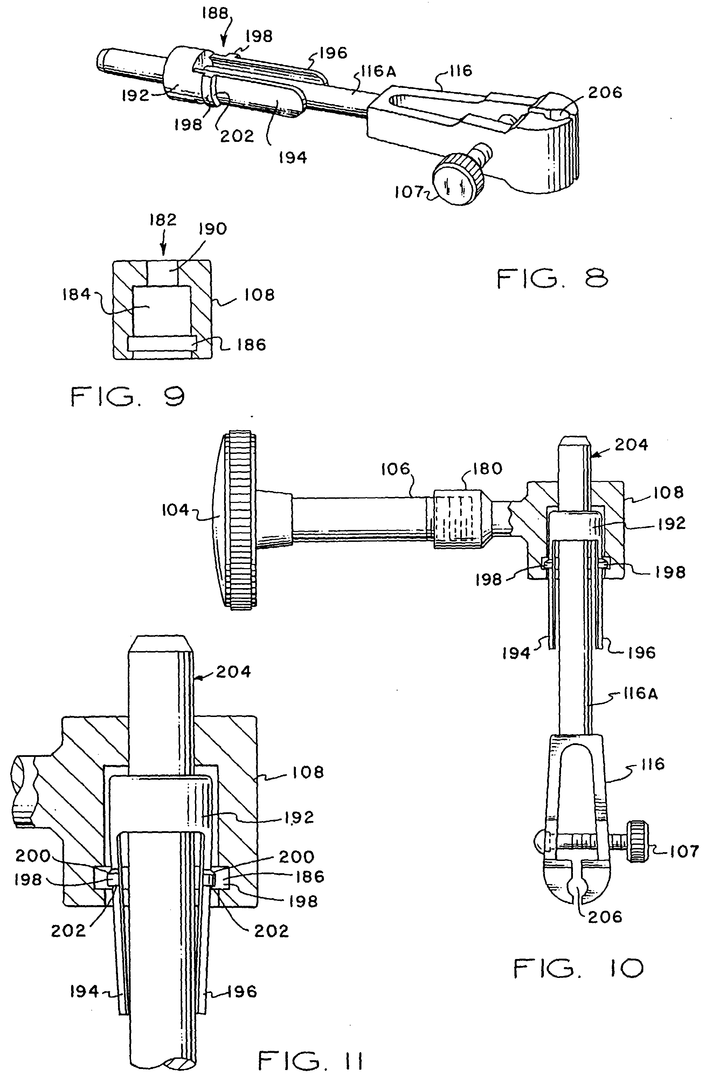

As shown in FIGS. 6 and 8, the coupling collar 108 includes a guide bore 182 extending from top to bottom along the axis F which is substantially perpendicular to the longitudinal axis E. The guide bore 182 is countersunk or counterbored, preferably about three-quarters along its length from the bottom, to form a retainer socket 184 having an inner sidewall. The inner sidewall 184 is further intersected by an annular detent groove 186 for receiving a quick release connector 188. A portion of the guide bore 182 which is not counterbored acts as a pilot guide 190 for guiding the distal end 204 of the shaft 116A through the coupling collar 108. The distal end 204 of the shaft 116A is preferably tapered for easy insertion into the pilot guide.

Referring now to FIG. 7, a quick release connector 188 is attached to the shaft 116A. The connector 188 is formed by a cylindrical collar 192 having axially projecting, resilient arms 194 and 196. The cylindrical collar 192 is secured to the shaft 116A with the resilient arms 194 and 196 projecting axially along the shaft 116A towards the clamp 116. The resilient arms 194 and 196 each have a rib or boss 198 in the general shape of an arc segment. Preferably, the boss 198 is chamfered on its top edge 200 to aid the connector 188 as it is inserted into the retainer socket 184. The boss 198 has a bottom edge 202 providing detended latched engagement within the annular groove 186, as shown in FIGURE 11.

The sterile clamp 116 preferably made of a disposable, reinforced resin, for example 25% glass-filled nylon or KEVLAR film polyester, or a reusable metallic material such as titanium or stainless steel. The clamp 116 is inserted through a preformed opening in the sterile drape 82 (FIGS. 1 and 2). The clamp 116 has an aperture 206 (FIG. 8 and FIG. 10) adjustable by a screw clamp 107 for accommodating a wide range of endoscope sizes/diameters. The clamp 116 is coupled to the coupling collar 118 by compressing or squeezing the resilient arms 194 and 196 inwardly and inserting the shaft 116A through the bottom of the collar 108 and into the bore 182. Compression of the resilient arms 194 and 196 permits each boss 198 to be inserted into the counterbore socket 184. With the shaft 116A inserted fully in the operative position as shown in FIG. 10, the clamp 116 may be freely rotated about the axis F (FIG. 6). After insertion, the resilient arms 194 and 196 are released, allowing them to expand or deflect radially outwardly, with each boss 198 being received in detented engagement within the annular groove 186. It is to be understood that while the preferred embodiment employs two arms 194 and 196, three or more arms may be used without departing from the scope of the present invention.

In the fully inserted, latched position, the distal end 204 of the shaft 116A projects out of the guide bore 190. The projecting end portion can be depressed by the surgeon S to apply a downward force in conjunction with an applied compressive force to the arms 194 and 196, to aid in withdrawing the clamp 116 from the collar 108. Preferably, the insert length (i.e. the distance from the distal end 204 of shaft 116A to the top of the inner sidewall 184) is equal or greater than the pilot length (i.e. the total length of the inner sidewall 184).

Upon completion of a surgical procedure, the clamp 116 is removed by compressing or squeezing the resilient arms 194 and 196 through the sterile drape 82. The articulated arm assembly 18 is then retracted, released and folded inwardly, and the video monitor 62 is retracted. The accessories, including the foot switch assembly 118 and signal conductor cable 130, are stored within the console compartments, and the glass doors 170, 172 are locked. The portable console assembly 10 is then ready for storage out of the operating area, and may be moved from one operating room to another.

## Claims

1. A surgical instrument holding apparatus comprising a clamp (116) for holding a surgical instrument (14), a shaft (116A) connected to the clamp, a coupling collar (108) having a latch bore (184) defining a retainer socket and including a latch receiver (186) in the retainer socket, and the shaft including a proximal end portion (204) and a distal end portion (116A), the proximal end portion being insertable into the retainer socket and movable to a latched position with the distal end portion projecting out of the retainer socket when the proximal end portion is in the latched position, and a manually operable latch (192) attached to the proximal end portion of the shaft for releasably connecting the shaft to the coupling collar, the latch including a latch head (198) engageable with the latch receiver (186) in the latched position, characterised in that:
the latch (192) includes at least two resilient actuator arms (194,196), each arm havina a latch head (198), which actuator arms are radially spaced from the shaft, an end portion of each actuator arm projecting out of the retainer socket (184) and being accessible for manual gripping outside of the coupling collar when the latch head is in the latched position, each actuator arm being movable radially inwardly relative to the shaft to permit the latch (192) to be manually inserted into and withdrawn from the retainer socket, and being movable radially outwardly relative to the shaft for moving the latch head (198) into latching engagement with the latch receiver (186).

2. A surgical instrument holding apparatus as claimed in Claim 1, wherein each latch head includes a latching boss or rib (198) engageable with the latch receiver (186) in the latched position.

3. A surgical instrument holding apparatus as claimed in claim 2, wherein each boss or rib (198) has a chamfered face (200) to aid insertion of the latch (192) into the retainer socket (184).

4. A surgical instrument holding apparatus as claimed in claim 2 or claim 3, wherein each boss or rib (198) has a flat face portion (202) disposed for interlocking engagement with the latch receiver (186) in the latched position.

5. A surgical instrument holding apparatus as claimed in any preceding claim, wherein the retainer socket (184) is bounded by a cylindrical sidewall, and the latch receiver (186) comprises an annual groove intersecting the cylindrical sidewall.

6. A surgical instrument holding apparatus as claimed in any preceding claim, wherein the latch head (198) comprises a rib or boss in the general shape of an arcuate segment.

7. A surgical instrument holding apparatus as claimed in any preceding claim, wherein the clamp (116) is made of stainless steel.

8. A surgical instrument holding apparatus as claimed in any preceding claim, wherein the coupling collar has a body portion (108) which is intersected by a guide bore (190), the guide bore defining a guide surface through the body portion, and the guide bore having a guide length which is less than the insert length of the shaft proximal end portion (204).

9. A surgical instrument holding apparatus as claimed in any preceding claim, wherein:
the coupling collar has a body portion (108) centrally disposed about a reference axis (F), the body portion being intersected by a guide bore (190) concentric with the latch bore (184) and extending along the reference axis, and the guide bore having a guide length less than the total length of the body portion; and,
the shaft (116A) including a pilot end portion (204) aligned with the reference axis and disposed in the guide bore (190) in the latched position.

10. A surgical instrument holding apparatus as claimed in claim 9, wherein the insert length of the pilot end portion (204) exceeds the guide length of the guide bore (190), wherein a portion of the pilot end portion projects out of the coupling collar (108) in the latched position.

11. A surgical instrument holding apparatus as claimed in claim 9 or claim 10, wherein the coupling collar (108) is intersected by a counterbore (184) and the guide bore (190) is intersected by a counterbore (184) and the guide bore (190) is in coaxial alignment with the counterbore, wherein the latch (192) is centred within the counterbore upon insertion of the proximal end (204) of the shaft into the guide bore.

12. A surgical instrument holding apparatus as claimed in any of claims 9 to 11, wherein the pilot end portion (204) of the shaft is tapered to aid insertion of the pilot end portion (204) into the guide bore (190).

13. A surgical instrument holding apparatus as claimed in any preceding claim, wherein:
the retainer socket (184) is bounded by a cylindrical sidewall extending partially through the coupling collar (108) and the cylindrical sidewall is intersected by a latch receiver groove (186); and,
the latch includes a pair of resilient actuator arms (194, 196), each arm having a latch head (198), which actuator arms project out of the latching socket (184) when the proximal end portion (204) of the shaft is in the latched position, the resilient actuator arms being manually deflectable radially inwardly relative to the shaft to permit the latch (192) to be inserted into and withdrawn from the retainer socket (184), and being radially deflectable outwardly for moving the latch head (198) into engagement with the latch receiver groove upon release thereof.

14. A surgical instrument holding apparatus as claimed in any preceding claim, wherein the latch includes an annular shoulder (192) and a pair of resilient actuator arms (194, 196), each arm having a latch head (198), which actuator arms depend therefrom in radially spaced relation to the shaft (116A), the actuator arms being accessible for manual gripping outside the coupling collar (108), and each actuator arm including an external boss or rib (198) for engaging the latch receiver (186).

15. A support assembly for positioning and releasably holding a surgical instrument; the support assembly including an articulated arm (18) adjustable in elevation relative to an operating table, the articulated arm including a proximal support arm section (20) and a distal support arm section (22), and a surgical instrument holding apparatus (188) releasably attached to the distal support arm section, characterised in that:
a surgical instrument holder (116) is coupled to the releasable instrument holding apparatus (188), the releasable instrument holding apparatus being constructed as set forth in any of claims 1 to 14.

## Patentansprüche

1. Vorrichtung zum Halten eines chirurgischen Instruments, die eine Klemme (116) zum Halten eines chirurgischen Instruments (14) umfasst sowie einen Schaft (116A), der mit der Klemme verbunden ist, eine Kupplungsmanschette (108) mit einer Einrastbohrung (184), die eine Haltemuffe definiert und einen Aufnehmer für die Einrastklinke (186) in der Haltemuffe einschließt, wobei der Schaft einen proximalen Endabschnitt (204) und einen distalen Endabschnitt (116A) einschließt, wobei der proximale Endabschnitt in die Haltemuffe eingeschoben und bis zu einer eingerasteten Stellung verschoben werden kann, wobei der distale Endabschnitt aus der Haltemuffe hervorsteht, wenn der proximale Endabschnitt in der eingerasteten Stellung vorliegt, sowie eine manuell betätigbare Einrastklinke (192), die an dem proximalen Endabschnitt des Schaftes befestigt ist, um den Schaft mit der Kupplungsmanschette lösbar zu verbinden, wobei die Einrastklinke einen Einrastkopf (198) einschließt, der in der eingerasteten Stellung mit dem Aufnehmer für die Einrastklinke (186) in Eingriff gebracht werden kann, dadurch gekennzeichnet, dass:
die Einrastklinke (192) wenigstens zwei federnde Stellarme (194, 196) einschließt, wobei jeder Arm einen Einrastkopf (198) aufweist, wobei die Stellarme radial vom dem Schaft beabstandet sind, sowie einen Endabschnitt jedes Stellarms, der aus der Haltemuffe (184) hervorsteht und zum manuellen Greifen außerhalb der Kupplungsmanschette zugänglich ist, wenn sich der Einrastkopf in der eingerasteten Stellung befindet, wobei jeder Stellarm radial nach innen relativ zu dem Schaft verschoben werden kann, damit die Einrastklinke (192) manuell in die Haltemuffe eingeschoben und von der Haltemuffe zurückgezogen werden kann, sowie radial nach außen relativ zu dem Schaft verschoben werden kann, um den Einrastkopf (198) in einen einrastenden Eingriff mit dem Aufnehmer für die Einrastklinke (186) zu verschieben.

2. Vorrichtung zum Halten eines chirurgischen Instruments nach Anspruch 1, wobei jeder Einrastkopf einen einrastenden Vorsprung oder eine einrastende Rippe (198) einschließt, der/die in der eingerasteten Stellung mit dem Aufnehmer für die Einrastklinke (186) in Eingriff gebracht werden kann.

3. Vorrichtung zum Halten eines chirurgischen Instruments nach Anspruch 2, wobei jeder Vorsprung oder jede Rippe (198) eine abgeschrägte Fläche (200) aufweist, um das Einschieben der Einrastklinke (192) in die Haltemuffe (184) zu unterstützen.

4. Vorrichtung zum Halten eines chirurgischen Instruments nach Anspruch 2 oder 3, wobei jeder Vorsprung oder jeder Rippe (198) einen ebenen Flächenabschnitt (202) aufweist, der zur verriegelnden In-Eingriffnahme mit dem Aufnehmer für die Einrastklinke (186) in der eingerasteten Stellung angeordnet ist.

5. Vorrichtung zum Halten eines chirurgischen Instruments nach einem der vorstehenden Ansprüche, wobei die Haltemuffe (184) durch eine zylindrische Seitenwand begrenzt wird und der Aufnehmer für die Einrastklinke (186) eine ringförmige Rinne umfasst, die die zylindrische Seitenwand unterteilt.

6. Vorrichtung zum Halten eines chirurgischen Instruments nach einem der vorstehenden Ansprüche, wobei der Einrastkopf (198) eine Rippe oder einen Vorsprung umfasst, die/der im wesentlichen die Form eines bogenförmigen Segments aufweist.

7. Vorrichtung zum Halten eines chirurgischen Instruments nach einem der vorstehenden Ansprüche, wobei die Klemme (116) aus nicht-rostendem Stahl hergestellt ist.

8. Vorrichtung zum Halten eines chirurgischen Instruments nach einem der vorstehenden Ansprüche, wobei die Kupplungsmanschette einen Gehäuseabschnitt (108) aufweist, der durch eine Führungsbohrung (190) unterteilt wird, wobei die Führungsbohrung eine Führungsfläche durch den Gehäuseabschnitt definiert und die Führungsbohrung eine Führungslänge aufweist, die geringer als die Einschublänge des proximalen Endabschnitts (204) des Schafts ist.

9. Vorrichtung zum Halten eines chirurgischen Instruments nach einem der vorstehenden Ansprüche, wobei:
die Kupplungsmanschette einen Gehäuseabschnitt (108) aufweist, der zentral um eine Referenzachse (F) angeordnet ist, wobei der Gehäuseabschnitt durch eine Führungsbohrung (190) unterteilt wird, die mit der Einrastbohrung (184) konzentrisch ist und sich entlang der Referenzachse ausdehnt, und wobei die Führungsbohrung eine Führungslänge aufweist, die geringer als die Gesamtlänge des Gehäuseabschnitts ist; und
der Schaft (116A) einen Einführ-Endabschnitt (204) einschließt, der mit der Referenzachse ausgerichtet und in der eingerasteten Stellung in der Führungsbohrung (190) angeordnet ist.

10. Vorrichtung zum Halten eines chirurgischen Instruments nach Anspruch 9, wobei die Einschublänge des Einführ-Endabschnitts (204) die Führungslänge der Führungsbohrung (190) übertrifft, wobei ein Abschnitt des Einführ-Endabschnitts in der eingerasteten Stellung aus der Kupplungsmanschette (108) hervorsteht.

11. Vorrichtung zum Halten eines chirurgischen Instruments nach Anspruch 9 oder 10, wobei die Kupplungsmanschette (108) durch eine zylindrische Senke (184) unterteilt ist und die Führungsbohrung (190) durch eine zylindrische Senke (184) unterteilt ist und die Führungsbohrung (190) in koaxialem Ausrichtung mit der zylindrischen Senke (184) ist, wobei die Einrastklinke (192) in der zylindrischen Senke (184) nach dem Einschieben des proximalen Endes (204) des Schafts in die Führungsbohrung zentriert wird.

12. Vorrichtung zum Halten eines chirurgischen Instruments nach einem der Ansprüche 9 bis 11, wobei der Einführ-Endabschnitt (204) des Schafts konisch zuläuft, um das Einschieben des Einführ-Endabschnittes (204) in die Führungsbohrung (190) zu unterstützen.

13. Vorrichtung zum Halten eines chirurgischen Instruments nach einem der vorstehenden Ansprüche, wobei:
die Haltemuffe (184) durch eine zylindrische Seitenwand begrenzt wird, die sich partiell in der Kupplungsmanschette (108) ausdehnt, und die zylindrische Seitenwand durch eine Aufnahmerinne für die Einrastklinke (186) unterteilt wird; und
die Einrastklinke ein Paar federnder Stellarme (194, 196) einschließt, wobei jeder Arm einen Einrastkopf (198) aufweist, und wobei die Stellarme aus der einrastenden Muffe (184) hervorstehen, wenn sich der proximale Endabschnitt (204) des Schafts in der eingerasteten Stellung befindet, wobei die federnden Stellarme manuell radial nach innen relativ zum Schaft abgebogen werden können, damit die Einrastklinke (192) in die Haltemuffe (184) eingeschoben und von der Haltemuffe zurückgezogen werden kann, sowie radial nach außen abgebogen werden können, um den Einrastkopf (198) in eine In-Eingriffnahme mit der Aufnahmerinne für die Einrastklinke (186) nach dessen Freigabe zu verschieben.

14. Vorrichtung zum Halten eines chirurgischen Instruments nach einem der vorstehenden Ansprüche, wobei die Einrastklinke eine ringförmige Schulter (192) und ein Paar federnder Stellarme (194, 196) einschließt, wobei jeder Arm einen Einrastkopf (198) aufweist, wobei die Stellarme davon in radial beabstandeter Beziehung zu dem Schaft (116A) abhängen, wobei die Stellarme zum manuellen Greifen außerhalb der Kupplungsmanschette (108) zugänglich sind, und wobei jeder Stellarm einen äußeren Vorsprung oder eine äußere Rippe (198) zum In-Eingriff-Nehmen mit dem Aufnehmer für die Einrastklinke (186) einschließt.

15. Eine Stützanordnung zum Positionieren und lösbaren Halten eines chirurgischen Instruments; wobei die Stützanordnung einen Gelenkarm (18) einschließt, der in der Höhe relativ zu einem Operationstisch anpassbar ist, wobei der Gelenkarm einen proximalen Stützarmabschnitt (20) und einen distalen Stützarmabschnitt (22) und eine Vorrichtung zum Halten eines chirurgischen Instruments (188) einschließt, die lösbar an dem distalen Stützarmabschnitt befestigt ist, dadurch gekennzeichnet, dass:
eine Halterung für ein chirurgisches Instrument (116) mit der Vorrichtung zum lösbaren Halten eines Instruments (188) gekoppelt ist, wobei die Vorrichtung zum lösbaren Halten eines Instruments gemäß den Ansprüchen 1 bis 14 ausgebildet ist.

## Revendications

1. Appareil de maintien d'un instrument chirurgical, comprenant une pince (116) destinée au maintien d'un instrument chirurgical (14), un arbre (116A) raccordé à la pince, un collier d'accouplement (108) ayant un trou (184) de verrouillage qui délimite un logement d'organe de retenue et comprenant un organe récepteur de verrou (186) dans le logement d'organe de retenue, et l'arbre comprenant une partie d'extrémité proximale (204) et une partie d'extrémité distale (116A), la partie d'extrémité proximale pouvant être insérée dans le logement d'organe de retenue et étant mobile vers une position verrouillée dans laquelle la partie d'extrémité distale dépasse à l'extérieur du logement d'organe de retenue lorsque la partie d'extrémité proximale est en position verrouillée, et un verrou (192) qui peut être manoeuvré manuellement, fixé à la partie d'extrémité proximale de l'arbre et destiné à raccorder temporairement l'arbre au collier d'accouplement, le verrou comprenant une tête de verrou (198) qui peut coopérer avec l'organe récepteur de verrou (186) en position verrouillée, caractérisé en ce que :
le verrou (192) comporte au moins deux bras élastiques (194, 196) d'organe de manoeuvre, chaque bras ayant une tête de verrou (198), les bras d'organe de manoeuvre étant espacés radialement par rapport à l'arbre, une partie d'extrémité de chaque bras d'organe de manoeuvre dépassant à l'extérieur du logement (184) d'organe de retenue et étant accessible pour être saisie manuellement à l'extérieur du collier d'accouplement lorsque la tête de verrou est en position verrouillée, chaque bras d'organe de manoeuvre étant mobile radialement vers l'intérieur par rapport à l'arbre pour permettre l'insertion manuelle du verrou (192) dans le logement d'organe de retenue et l'extraction du verrou de ce logement, et étant mobile radialement vers l'extérieur par rapport à l'arbre afin que la tête de verrou (198) soit déplacée en coopération de verrouillage avec l'organe récepteur de verrou (186).

2. Appareil de maintien d'un instrument chirurgical selon la revendication 1, dans lequel chaque tête de verrou comprend une nervure ou saillie de verrouillage (198) destinée à coopérer avec l'organe récepteur de verrou (186) dans la position verrouillée.

3. Appareil de maintien d'un instrument chirurgical selon la revendication 2, dans lequel chaque nervure ou saillie (198) a une face chanfreinée (200) destinée à faciliter l'insertion du verrou (192) dans le logement d'organe de retenue (184).

4. Appareil de maintien d'un instrument chirurgical selon la revendication 2 ou 3, dans lequel chaque nervure ou saillie (198) a une partie de surface plate (202) disposée afin qu'elle coopère par emboîtement avec l'organe récepteur de verrou (186) en position verrouillée.

5. Appareil de maintien d'un instrument chirurgical selon l'une quelconque des revendications précédentes, dans lequel le logement d'organe de retenue (184) est délimité par une paroi latérale cylindrique, et l'organe récepteur de verrou (186) comporte une gorge annulaire recoupant la paroi latérale cylindrique.

6. Appareil de maintien d'un instrument chirurgical selon l'une quelconque des revendications précédentes, dans lequel la tête de verrou (198) comporte une nervure ou saillie ayant la forme générale d'un segment courbe.

7. Appareil de maintien d'un instrument chirurgical selon l'une quelconque des revendications précédentes, dans lequel la pince (116) est formée d'acier inoxydable.

8. Appareil de maintien d'un instrument chirurgical selon l'une quelconque des revendications précédentes, dans lequel le collier d'accouplement a une partie de corps (108) qui est recoupée par un trou de guidage (190), le trou de guidage délimitant une surface de guidage dans la partie de corps et le trou de guidage ayant une longueur de guidage inférieure à la longueur d'insertion de la partie d'extrémité proximale (204) de l'arbre.

9. Appareil de maintien d'un instrument chirurgical selon l'une quelconque des revendications précédentes, dans lequel :
le collier d'accouplement a une partie de corps (108) placée au centre autour d'un axe de référence (F), la partie de corps étant recoupée par un trou de guidage (190) concentrique au trou de verrou (184) et s'étendant le long de l'axe de référence, le trou de guidage ayant une longueur de guidage inférieure à la longueur totale de la partie de corps, et
l'arbre (116A) comporte une partie d'extrémité de pilotage (204) alignée sur l'axe de référence et disposée dans le trou de guidage (190) en position verrouillée.

10. Appareil de maintien d'un instrument chirurgical selon la revendication 9, dans lequel la longueur d'insertion de la partie d'extrémité de pilotage (204) dépasse la longueur de guidage du trou de guidage (190), une partie de la partie d'extrémité de pilotage dépassant à l'extérieur du collier d'accouplement (108) dans la position verrouillée.

11. Appareil de maintien d'un instrument chirurgical selon la revendication 9 ou 10, dans lequel le collier d'accouplement (108) est recoupé par un alésage (184) et le trou de guidage (190) est recoupé par un alésage (184) et le trou de guidage (190) est aligné coaxialement sur l'alésage, et le verrou (192) est centré dans l'alésage après insertion de l'extrémité proximale (204) de l'arbre dans le trou de guidage.

12. Appareil de maintien d'un instrument chirurgical selon l'une quelconque des revendications 9 à 11, dans lequel la partie d'extrémité de pilotage (204) de l'arbre est effilée afin que l'insertion de la partie d'extrémité de pilotage (204) dans le trou de guidage (190) soit facile.

13. Appareil de maintien d'un instrument chirurgical selon l'une quelconque des revendications précédentes, dans lequel :
le logement d'organe de retenue (184) est délimité par une paroi latérale cylindrique qui s'étend partiellement autour du collier d'accouplement (108) et la paroi latérale cylindrique est recoupée par une gorge (186) d'organe de logement de verrou, et
le verrou comprend deux bras élastiques d'organe de manoeuvre (194, 196), chaque bras ayant une tête de verrou (198), les bras d'organe de manoeuvre dépassant du logement de verrouillage (184) lorsque la partie d'extrémité proximale (204) de l'arbre est en position verrouillée, les bras élastiques d'organe de manoeuvre pouvant fléchir manuellement en direction radiale vers l'intérieur par rapport à l'arbre pour permettre l'insertion du verrou (192) dans le logement d'organe de retenue (184) et son extraction de ce logement, et pouvant fléchir radialement vers l'extérieur afin que la tête de verrou (198) soit déplacée au contact de la gorge d'organe récepteur de verrou après libération de la tête.

14. Appareil de maintien d'un instrument chirurgical selon l'une quelconque des revendications précédentes, dans lequel le verrou comprend un épaulement annulaire (192) et deux bras élastiques d'organe de manoeuvre (194, 196), chaque bras ayant une tête de verrou (198), les bras d'organe de manoeuvre dépassant sous cette tête à des emplacements radialement espacés vers l'arbre (116A), les bras d'organe de manoeuvre étant accessibles pour une saisie manuelle à l'extérieur du collier d'accouplement (108), chaque bras d'organe de manoeuvre comportant une nervure ou saillie externe (198) destinée à coopérer avec l'organe récepteur de verrou (186).

15. Ensemble de support destiné au positionnement et au maintien temporaire d'un instrument chirurgical, l'ensemble de support comprenant un bras articulé (18) réglable en hauteur par rapport à une table d'opération, le bras articulé comportant un tronçon de bras de support proximal (20) et un tronçon de bras de support distal (22), et un appareil (188) de maintien d'instrument chirurgical fixé de façon temporaire au tronçon de bras de support distal, caractérisé en ce que :
un organe (116) de maintien d'instrument chirurgical est couplé à l'appareil (188) de maintien d'instrument de façon amovible, l'appareil de maintien d'instrument de façon amovible ayant une construction selon l'une quelconque des revendications 1 à 14.
